# EUROPEAN PATENT APPLICATION

(11) **EP 4 223 781 A1**
(43) Date of publication of application: **09.08.2023**
(21) Application number: 22155256.5
(22) Date of filing: 04.02.2022
(51) Int. Cl.: C07K 16/28, G01N 33/53

(54) **NOVEL DESMOGLEIN 3 (DSG3)-EC5-SPECIFIC MONOCLONAL ANTIBODY 2G4**

(71) Applicant: PHILIPPS-UNIVERSITÄT MARBURG, 35037 Marburg (DE)
(72) Inventor: Eming, Rüdiger, 56075 Koblenz (DE); Hudemann, Christoph, 34131 Kassel (DE); Hertl, Michael, 35039 Marburg (DE)
(74) Representative: Durm Patentanwälte PartG mbB

(57) **Abstract**

Disclosed is a monoclonal IgG antibody 2G4 which
• is specific for the membrane-proximal extracellular region EC5 of Desmoglein-3 designated as human Dsg3-EC5 (aa451-566)
• shows no cross-reaction with mouse Dsg3 or human Dsg1
• is capable of inhibiting the homophilic Dsg3-trans-interaction
• induces cellular Dsg3 redistribution by activation of the p38MAPK signal transduction pathway and
• exhibits pathogenic Ca2+-independent binding to human Dsg3.

The B-cell hybridoma producing said novel antibody 2G4 has been deposited at DSMZ Braunschweig on January 11, 2022 under accession number DSM ACC3371.

The novel antibody 2G4 can be used as an *in vitro* diagnostic for pemphigus vulgaris and as a scientific tool for analyzing the molecular and cellular mechanisms involved in pemphigus vulgaris.

## Description

The present invention relates to the diagnosis of pemphigus vulgaris. In particular, the invention provides a new desmoglein 3 (Dsg3)-EC5-specific monoclonal antibody 2G4, preferably the monoclonal antibody produced by a B-cell hybridoma cell line registered at the DSMZ under the accession number DSM ACC3371. The antibody may be useful as an *in vitro* diagnostic for pemphigus vulgaris, in particular a positive control reagent, and as a scientific tool for elucidating the molecular and/or cellular mechanisms involved in pemphigus vulgaris, a process for obtaining said novel antibody and the use of the antibody as an *in vitro* diagnostic, as well as the use of the antibody as a scientific tool.

### Background of the invention

Pemphigus vulgaris (PV) is a severe autoimmune blistering disease characterized by IgG autoantibodies (auto-ab) against the desmosomal adhesion molecules desmoglein 3 (Dsg3) and Dsg1. Underlying mechanisms leading to blister formation upon binding of Dsg-specific IgG auto-ab are not fully understood. Numerous studies demonstrated the pathogenicity of IgG auto-ab binding to the amino-terminal region 1 (EC1) of the Dsg3 ectodomain. However, auto-ab in PV are polyclonal, including IgG against both amino-and membrane proximal epitopes of the Dsg3 ectodomain.

In more detail, pemphigus vulgaris (PV) is a potentially lethal IgG autoantibody (auto-ab) driven autoimmune disorder involving mucous membranes and the skin. A hallmark of PV is the presence of IgG auto-ab against distinct desmosomal proteins of the epidermis, leading to a loss of keratinocyte adhesion (i.e. acantholysis). Such acantholysis induces the typical clinical phenotype with flaccid blisters and erosions. The desmosomal cadherins, desmoglein 1 (Dsg1) and Dsg3, are the major autoantigens targeted by the circulating auto-ab.

PV can be divided in two major subtypes, depending on the clinical manifestation and the auto-ab profile. Dsg1 auto-ab induce superficial blister formation, clinically named pemphigus foliaceus. In PV, Dsg3 represents the central autoantigen in the pathogenesis of the disease. Due to the differential expression of Dsg1 and Dsg3, Dsg1-specific auto-ab preferentially bind to desmosomes in the upper epidermis, whereas Dsg3-specific antibodies induce acantholysis in the basal and suprabasilar layers.

Although PV is a relatively well-analysed autoimmune disease, molecular mechanisms that induce the loss of desmosomal function are still controversial. Two major concepts are favoured: 1) a direct impact of the Dsg3 auto-ab on the desmosomal structure via steric hindrance, or 2) auto-ab inducing various cell signalling events resulting in the loss of the desmosomal structure.

Previous publications suggest a connection of auto-ab binding to Dsg3, the activation of the p38 mitogen-activated protein kinase (MAPK), epidermal growth factor receptor (EGFR) and c-Myc signalling pathways and acantholysis, but the underlying molecular mechanisms are still unclear. The p38 MAPK pathway is the best examined component in this context, but recent data are also inconsistent. There is evidence for p38 activation after binding of PV auto-ab in a PV animal model, using PV patient skin and in vitro assays using the keratinocyte cell line HaCaT. Still, mice lacking the major p38 MAPK isoforms exhibit blister formation, suggesting involvement of p38 independent pathways.

Additionally, studies indicate endocytosis of Dsg3 after auto-ab binding and the activation of apoptotic pathways, leading to the detachment of keratinocytes.

Furthermore, cadherin-independent auto-ab can be detected in PV sera and may represent a not yet fully understood component contributing to this complex process.

Like other cadherins, Dsg3 and Dsg1 consist of an extracellular domain, a transmembrane domain and an intracellular part, respectively. The extracellular portion mediates calcium (Ca²⁺) dependent homophilic binding between Dsg3 molecules, representing the central mechanism for the maintenance of desmosomal integrity. Additionally, there are critical heterophilic interactions between Dsg3 and desmocollins, which strengthen the cohesion of the desmosomal cell-cell contacts. In vitro, Ca²⁺ concentrations over 0.1 mM rapidly induce cell adhesion of HaCaT keratinocytes and formation of functional desmosomes within 5 h, whereas Ca²⁺ concentrations below 0.1 mM prevent desmosome formation.

The extracellular domain of Dsg3, similar to other cadherins, consists of five subdomains (EC1-EC5) that share a high structural homology, except for the COOH-terminal EC5 domain (Koch P. J. et al., "Identification of desmoglein, a constitutive desmosomal glycoprotein, as a member of the cadherin family of cell adhesion molecules", European journal of cell biology 1990;53(1):1-12).

Numerous studies have shown that most pemphigus-specific auto-ab bind to the N-terminal EC1-subdomain, the major mediator of homo- and heterophilic interactions.

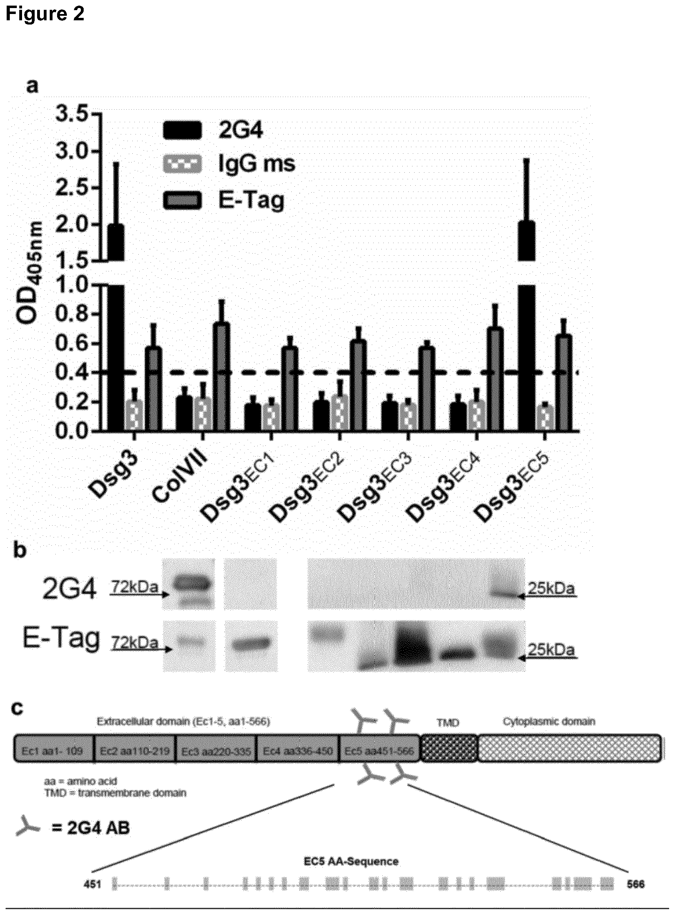

The EC5 domain is shown in Fig. 2c and consists of amino acid residues 451 to 566 of Dsg3. The sequence of Dsg3-EC5 is given in SEQ ID NO: 1.

SEQ ID NO: 2 shows the complete extracellular domain of Dsg3.

Approximately 50 % of PV patients exhibit serum IgG directed against the EC1 domain, followed by EC2 (26 %), EC4 (21 %), EC5 (17 %) and EC3 (15 %). Due to their high prevalence in PV patients and their high pathogenicity in several in vitro and in vivo studies, EC1-specific antibodies, such as the mouse monoclonal IgG antibody Ak23, are typically used for investigating PV pathology.

The impact of IgG targeting other Dsg3 subdomains on keratinocyte signalling and desmosomal structure, which might deliver new aspects for the understanding of the acantholytic process, has so far not been studied in detail.

On this background, the problem of the invention is to provide a novel monoclonal antibody which is specific for the EC5 domain of Dsg3, in order to provide a novel diagnostic for pemphigus vulgaris and a scientific tool for elucidating the molecular and cellular mechanisms involved in pemphigus vulgaris.

### Summary of the invention

The problem has been solved by the monoclonal IgG antibody 2G4 defined in claims 1, 2 and 4.

The invention further provides a process for obtaining said antibody 2G4, an *in vitro* diagnostic use of said antibody and the use of said antibody as a scientific tool, as defined in the claims.

The inventors thoroughly investigated the inhibitory impact of a novel EC5-directed monoclonal IgG antibody 2G4 on keratinocyte adhesion. They found that said antibody 2G4 induces loss of keratinocyte adhesion, comparable to AK23, that involves downstream activation of p38 MAPK.

The inventors have studied, characterized and tested the pathogenicity of the claimed monoclonal IgG antibody directed against the membrane-proximal region (EC5) of the Dsg3 ectodomain, 2G4, in various specificity and functional assays.

The results clearly demonstrate that the novel antibody 2G4 is capable of inhibiting intercellular keratinocyte adhesion, inducing cellular Dsg3 redistribution by activation of the p38MAPK signal transduction pathway.

The inventors provide, for the first time, evidence that an IgG auto-ab directed against the membrane-proximal region EC5 of Dsg3 is pathogenic *in vivo.* These findings are contrary to the current concept that only IgG auto-ab targeting the NH₂-terminal portion of the Dsg3 ectodomain are pathogenic.

### Detailed description of the invention

### Monoclonal antibody 2G4 binds specifically to human Dsg3 protein in a Calcium-independent manner

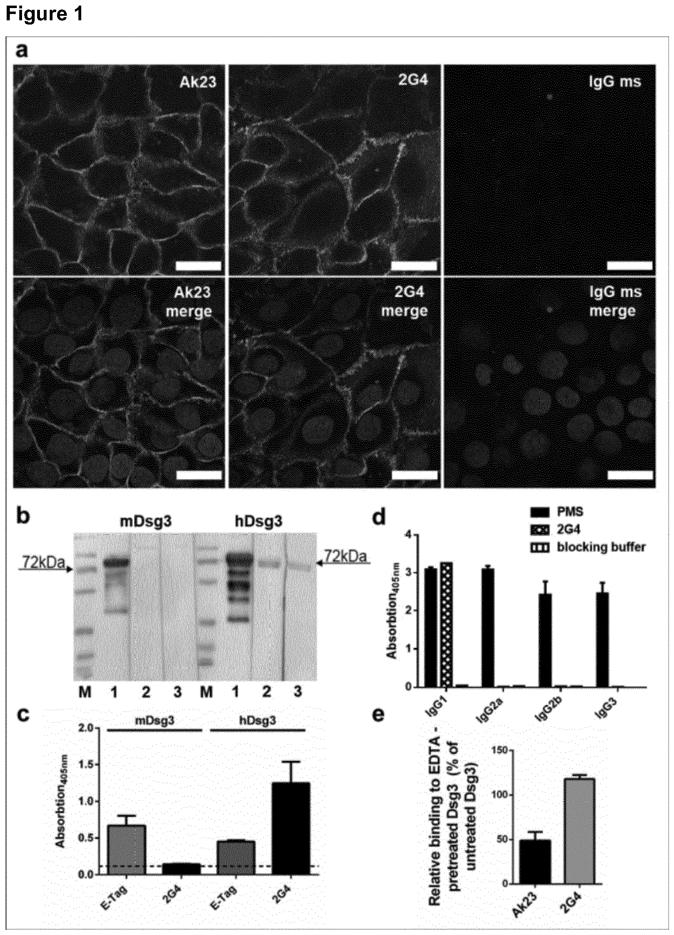

To test whether the antibody of the invention, 2G4, targets human Dsg3, immunofluorescence (IF), ELISA and Western blot analysis were used. In IF microscopy, the 2G4 antibody staining showed the fence-like cell surface staining pattern characteristic of Dsg3 and comparable to the known EC1-specific monoclonal antibody AK23. The mouse IgG control (IgG ms) did not show a specific staining pattern (Figure 1a).

ELISA and Western blot analysis showed that 2G4 is specific for human Desmoglein 3, without cross-reactivity with murine Dsg3 (Figure 1b, c) or human Desmoglein 1 (not shown). The IgG1 subtype of 2G4 was verified by ELISA (Figure 1d).

As already indicated by binding to denatured Dsg3 in Western blot, Ca²⁺-independent binding of 2G4 to Dsg3 pre-treated with ethylenediaminetetraacetic acid (EDTA) was confirmed by ELISA.

In contrast to AK23 with a Ca²⁺-dependent, conformational epitope, the inventors' findings revealed that 2G4 binds to Dsg3 in a Ca²⁺-independent manner (Figure 1e).

### 2G4 specifically binds to a membrane-proximal epitope of Dsg3

To further analyse the Dsg3 epitope that is targeted by the novel antibody 2G4 of the invention, ELISA and Western blot analysis were performed with the complete ectodomain of human Dsg3, Dsg3 subdomains EC1 to EC5 and the collagen VII NC 1 domain (control).

In ELISA, 2G4 clearly reacted with the membrane-proximal part of Dsg3, the subdomain EC5, and the full ectodomain of Dsg3 (Figure 2a). The optical density at 40 nm is shown.

Western blot analyses including E-Tag staining and collagen VII NC1 domain confirmed the ELISA results. Under denaturing and reducing conditions, 2G4 specifically bound to the full-length Dsg3 ectodomain and EC5 subdomain, but not to the other subdomains of human Dsg3 (Figure 2b). Thus, 2G4 specifically targets the membrane-proximal EC5 domain of human Dsg3, as summarized in Figure 2c.

### 2G4 inhibits Dsg3 trans-interaction and induces activation of the p38 MAPK signalling

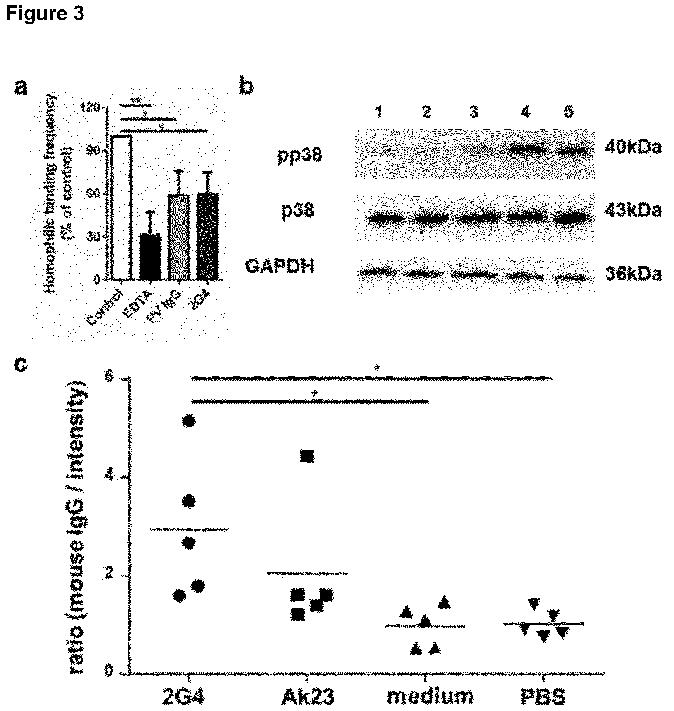

The ability of the novel inventive antibody 2G4 to disturb homophilic Dsg3-Dsg3 binding was analysed using a cell-free system based on atomic force microscopy (AFM). Incubation of HaCaT cells with 2G4 led to a substantial decrease of the homophilic Dsg3 binding events, comparable to the purified PV IgG pool. 2G4 is able to induce pathogenic effects in vitro, similar to PV IgG the response of which is often dominated by Dsg3 - EC1 specific IgG (Figure 3a).

To test the ability of 2G4 to activate the p38MAPK signal transduction pathway, 2G4 was added to confluent HaCaT cells that were then lysed, and p38MAPK, phosphorylated p38MAPK (pp38) and the house-keeping protein, glyceraldehyde 3-phosphate dehydrogenase (GAPDH), were analysed by Western blot (Figure 3b). Pooled results of experiments including mouse IgG and Ak23 are presented in Figure 3c. To exclude inter-assay variations, the quantitative values were defined as the signal intensity normalized to GAPDH. Values are presented relative to the negative control (mouse IgG). Phosphorylated p38MAPK also serves as a sensitive marker for cell stress. Thus, there is always a baseline signal for pp38MAPK upon addition of PBS, cell culture media and mouse IgG. However, the amount of pp38MAPK was markedly increased after stimulation with 2G4 or Ak23, demonstrating that binding of 2G4 to Dsg3 in cultured keratinocytes leads to phosphorylation of p38MAPK.

Furthermore, a Dsg3-reactive antibody against the membrane-proximal EC5-domain is able to induce activation of signal transduction pathways in cultured keratinocytes, similarly to EC1-specific IgG.

### 2G4 causes a marked change in Dsg3 distribution in epidermal keratinocytes

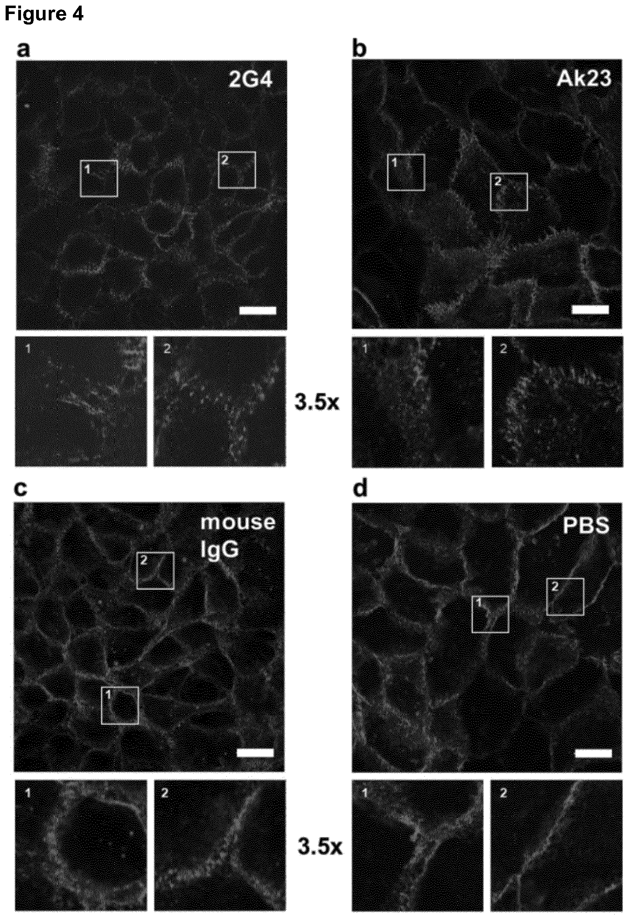

To determine the effects of the novel 2G4 of the invention on the desmosomal structure, the distribution and localization of Dsg3 in cultured HaCaT cells after 2G4 treatment was analysed by confocal microscopy. After 2G4 incubation, Dsg3 distribution changed from the regular fence-like pattern to a more diffuse and chain-like structure, similar to the effects of Ak23 and in contrast to PBS and mouse IgG (Figure 4). Although the keratinocytes were still forming a monolayer, the cell-cell contact areas were markedly affected (Figure 4a, Zoom 1). Additionally, after incubation of HaCaT cells with 2G4, Dsg3 was located in structures resembling budding vesicles, suggesting an ongoing endocytosis (Figure 4a, Zoom 2).

### 2G4 induces loss of keratinocyte adhesion in vitro and ex vivo

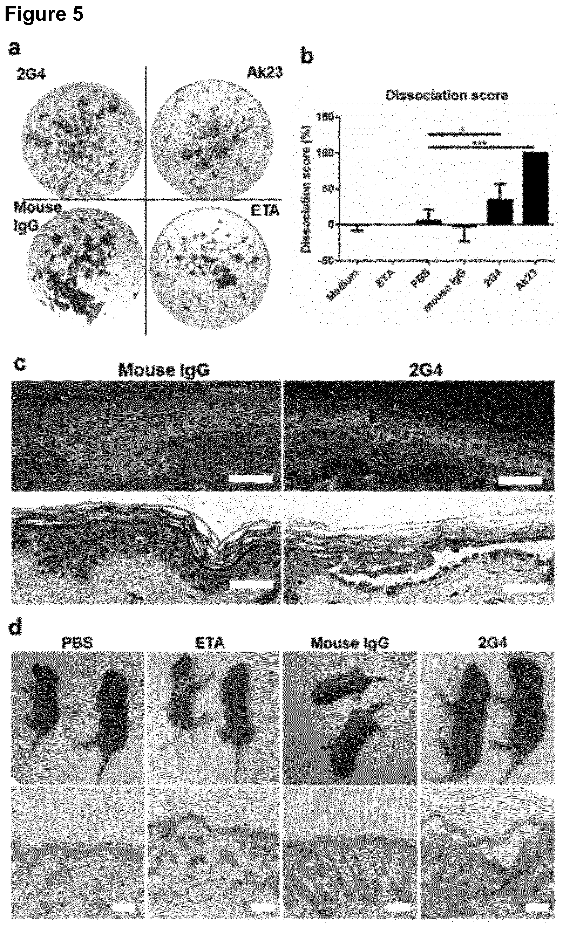

Although the cohesion of the keratinocyte monolayer seemed largely unaffected by the novel antibody 2G4 of the invention, changes in the desmosomal structure evidenced by altered Dsg3 distribution (Figure 4) indicated an inhibitory impact on desmosomal integrity. To investigate a potential loss of desomosomal adhesion by 2G4, a dispase-based dissociation assay (DBD-assay) was performed by subjecting a HaCaT monolayer to shear forces. Reduced desmosomal integrity leads to higher number of monolayer fragments. After 2G4 treatment, keratinocyte monolayers dissociated into a significantly higher number of fragments compared to IgG control (Figure 5a), clearly indicating a disruptive impact of 2G4 on the desmosomal integrity (Figure 5b).

To further elucidate these findings, the influence of 2G4 on intercellular adhesion of keratinocytes in healthy human skin was analysed by injection into the epidermis. As depicted in Figure 5c, 2G4 was able to specifically bind to the cell surface of epidermal keratinocytes. Furthermore, it induced an intra-epidermal blister formation at the suprabasal layer, typical for pathogenic anti-Dsg3 antibodies.

For the characterization of 2G4 in vivo, the inventors employed a mouse model bearing a chimeric Dsg3 with human Dsg3 extracellular domain fused to a murine transmembrane and intracellular domain. Compared to respective controls, passive transfer of 2G4 induced a pronounced blister formation.

To summarize, similar to the Dsg3-EC1 specific IgG antibody Ak23, the EC5-specific 2G4 IgG antibody exhibits a strong pathogenic activity on keratinocyte adhesion both in vitro and in vivo (see Table 1 below).

### Description of Figures

Fig. 1 shows that the novel monoclonal IgG antibody 2G4 of the invention binds to human Dsg3.
Fig. 1a: IF shows binding of 2G4 to the cell surface of human keratinocytes (HaCaT).
Fig. 1b: Specificity of 2G4 for human Dsg3 shown by Western blot. 1 = E-tag, 2 = 2G4 (60µg/ml), 3 = 2G4 (30µg/ml).
Fig.1 c: 2G4 binds exclusively to human Dsg3 protein. Similar proteins as for Western blots were used in ELISA; E-tag serves as positive control (dotted line = cut off level) (n=5).
Fig. 1d: Subtype specific ELISA. 2G4 (final 4µg/ml) and IgG1 (0.25 µg/ml), IgG2a (0.5 µg/ml), IgG2b (0.025 µg/ml) and IgG3 (0.25µg/ml); and blocking buffer as negative control. n=2 different experiments in triplicates.
Fig. 1e: Comparison of conventional and EDTA-treated ELISA shows that 2G4 recognizes a calcium (Ca²⁺) independent epitope. Displayed is reactivity of both antibodies compared to their non-EDTA-treated ELISA values set as 100% (n=3). Scale bar = 20 µm
Fig. 2 shows that the novel antibody 2G4 of the invention specifically binds to proximal membrane epitopes of the Dsg3 ectodomain.
Fig. 2a: Extracellular (EC)-specific ELISA confirms EC5 specificity. Recombinant Dsg3 (extracellular domain), Dsg3-subdomains (EC1-5) and ColVII as negative control were analysed. 2G4, anti E-tag and murine IgG served as primary antibodies. (n = 4).
Fig. 2b:EC-specific Western blot confirms EC5 specificity under reducing conditions. Similar proteins and antibodies as described under (Fig. 2a) were used. 2G4 binds only to the EC5 subdomain and the Dsg3 ectodomain (extracellular domain).
Fig. 2c: Schematic view of Dsg3 protein. The extracellular domain (EC domain) of Dsg3 (aa 1-566) is divided into five subdomains (EC1-EC5). 2G4 specifically binds to the membrane proximal EC5 domain (aa 451-566).
Fig. 2d: Scheme displaying the amino acid sequence of the EC5 domain of Dsg3. Light grey marked regions show parts with difference to mouse Dsg3 and possible 2G4 binding sides.
Fig. 3 shows that the novel antibody 2G4 of the invention inhibits Dsg3 trans-interaction and activates cell signalling pathways.
Fig. 3a: The blocking effect of 2G4 on Dsg3-Dsg3 interaction is comparable to PV-IgG.
Fig. 3b: Activation of p38 mitogen-activated protein kinase (MAPK) by 2G4. Lysed HaCaT cells were analysed for p38 phosphorylation after stimulation with medium control (#1), phosphate-buffered saline (PBS) (#2), mouse IgG (100 µg/ml, negative control) (#3), 2G4 (100 µg/ml) (#4) and Ak23 (25 µg/ml, positive control) (#5). One representative experiment of five is shown.
Fig. 3c: Western blot intensity values of phosphorylated p38 were normalized to GAPDH. The diagram shows the factor of activity compared to mouse IgG, bars show the mean. Both 2G4 and Ak23 can activate p38, 2G4 3.5 times higher than PBS or medium, (n =5). A Mann-Whitney-Test with non-adjusted p-value was performed. * p≤0.05, ** p≤0.01, *** p≤0.001
Fig. 4 shows that binding of the novel antibody 2G4 of the invention causes changes in cellular Dsg3 distribution. The effect of antibody binding on HaCaT cells was analysed. Cells were grown to 70% confluence and subsequently treated with 2G4 (100µg/ml), Ak23 (25µg/ml), mouse IgG (100µg/ml) or PBS for 24 hours. Shown are representative images of one of three experiments.
Fig. 4a,b: 2G4 causes changes in Dsg3 distribution comparable to Ak23 (positive control). The Dsg3 pattern is still visible but it is clearly disrupted. The higher magnification (Factor 3.5) shows that cell-cell contacts are getting affected and Dsg3 might undergo endocytosis (Zoom 1 and 2).
Fig. 4c,d: Negative controls show no changes in Dsg3 pattern and the cell membrane does not show any alterations.
Fig. 5 demonstrates that 2G4 leads to disruption of desmosomes and split formation.
Fig. 5 a,b: Dispase based dissociation (DBD) assay using HaCaT cells treated with 2G4 (100 µg/ml), Ak23 (25 µg/ml), mouse IgG (100 µg/ml) and ETA (exfoliative toxin A, 0.5 µg/ml).
   (a) representative images from one of five independent DBD assays.
   (b) Dissociation score for respective antibodies, score = ((N_{ab}- N_{ETA})/(N_{Ak23}-N_{ETA}))*100, (N = amount of fragments). Ak23 serves as positive control set as 100%. Incubation with 2G4 significantly affects monolayer stability compared to negative controls (n = 5).
Fig. 5 c: Human skin biopsies either treated with mouse IgG (neg control) or 2G4 (200µg/ml) and 0.5µg/ml ETA.
Fig. 5d: Passive transfer of 2G4 into neonatal mice induced acantholysis *in vivo.* Scale bar = 50 µm. * p≤0.05, ** p≤0.01, *** p≤0.001

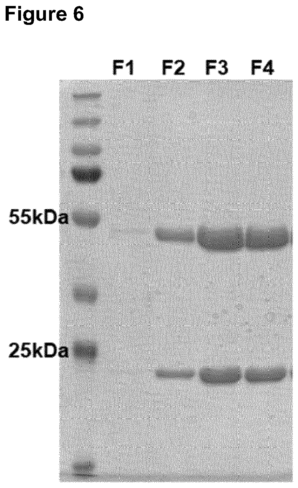

Fig. 6 shows 2G4 antibody purification. Exemplified elution (fraction F1-4) analysis of 2G4 after chromatography purification using Coomassie staining.

### Detailed description of the invention

PV sera contain a polyclonal mixture of Dsg3-specific IgG that represent a powerful tool to analyse PV associated pathological events, such as loss of desmosomal integrity and blister formation *in vitro* and *in vivo.* IgG auto-ab targeting the amino-terminal region of Dsg3 play a major role in the loss of cell-cell adhesion in PV, because this subdomain is critical for the homophilic interactions of adjacent desmosomal Dsg3 molecules. It has been shown that binding of such auto-ab leads to the loss of Dsg3 interaction. Nevertheless, EC1-specific monoclonal antibodies alone are less efficient in inducing loss of keratinocyte adhesion than polyclonal PV IgG.

Thus, an interplay of auto-ab directed against epitopes in other subdomains is necessary to develop the complete pathological potential of PV IgG.

The inventors provide evidence for the first time that binding of the novel monoclonal Dsg3 antibody 2G4 of the invention, which is specific for the Dsg3 EC5 domain, leads to loss of epidermal adhesion *ex vivo* and *in vivo,* challenging the concept that IgG directed against the EC1 subdomain alone are pathogenic.

To elucidate the impact of EC specificity on the pathological potential, numerous Dsg3 specific monoclonal antibodies have already been created.

Most of the EC1-specific antibodies such as Ak23 or AK19 (targeting peptides between EC1-and EC2) are pathogenic and bind conformational Dsg3-epitopes in a Ca²⁺-dependent manner.

Other monoclonal antibodies like 5G11 (EC1-EC2), Ak15 (EC2), Ak18 (EC3), 5H10 (EC4-EC5), Ak7 and Ak9 (both EC5) are Ca²⁺-independent but non-pathogenic (Kawasaki Y. et al., "Pathogenic monoclonal antibody against desmoglein 3 augments desmoglein 3 and p38 MAPK phosphorylation in human squamous carcinoma cell line", Autoimmunity 2006;39(7):587-90; Wahl J. K. "Generation of monoclonal antibodies specific for desmoglein family members". Hybridoma and hybridomics 2002;21(1):37-44; Tsunoda, K. et al., "Induction of Pemphigus Phenotype by a Mouse Monoclonal Antiboy Against the Amino-Terminal Adhesive Interface of Desmoglein 3", J Immunol 2003, 170 (4), 2170-2178, https://www.jimmunol.org/content/170/4/2170.long).

So far, EC1-specificity and the need for Ca²⁺ for a successful binding have been considered as indicators for a high pathogenicity.

The inventors here provide a novel monoclonal antibody targeting the membrane-proximal EC5 domain of Dsg3 that exhibits pathogenic effects similar to EC1-specific antibodies. In contrast to other pathogenic antibodies, such as Ak23, and similar to the pathogenic human monoclonal EC1-specific antibody PVMAB786, 2G4 epitope interaction takes place in a Ca²⁺ independent manner, indicative of a linear epitope.

2G4 was produced according to the invention by a monoclonal B-cell hybridoma cell line generated from a pool of murine splenocytes after immunization of an HLA-tg mouse model with the extracellular domain of human Dsg3 (Eming R. et al. "Pathogenic IgG antibodies against desmoglein 3 in pemphigus vulgaris are regulated by HLA-DRB1*04:02-restricted T cells", The Journal of Immunology 2014;193(9):4391-9). The B-cell hybridome producing the 2G4 antibody of the invention has been deposited under the Budapest treaty at DSMZ Braunschweig on January 11, 2022 and has been assigned the accession number DSM ACC3371.

This might be the reason why, in contrast to other Dgs3 specific monoclonal antibodies like AK23, the inventive 2G4 does not cross-react with murine Dsg3. Among the extracellular subdomains of human Dsg3, the membrane-proximal EC5 domain shares the lowest homology to murine Dsg3 (Tsunoda K. et al. "Induction of pemphigus phenotype by a mouse monoclonal antibody against the amino-terminal adhesive interface of desmoglein 3", Journal of immunology (Baltimore, Md. 1950) 2003;170(4):2170-8.). Because there is no cross-reactivity with mouse Dsg3, the potential epitopes of 2G4 are restricted to regions that are unique for human Dsg3 (Figure 2b).

One central component in the loss of human keratinocyte adhesion is the disturbance of intradesmosomal Dsg3 organization after antibody binding, but whether a single antibody related cell-cell disruption event is sufficient, remains elusive. For the stabilization of the desmosomal structure, cadherins undergo homophilic and heterophilic interactions. At the molecular level, Dsg3 proteins of adherent keratinocytes exhibit the potential to interact with each other. In a cell-free system, it was demonstrated that PV IgG directly inhibits Dsg3 mediated trans-interaction. Thus, loss of Dsg3-Dsg3 interplay after antibody binding directly indicates its pathogenicity. The homophilic binding is mainly based on the amino-terminal EC1 domains of the Dsg3 protein, and the EC1 specific antibodies have thus been intensively investigated. They induce inhibition mainly by steric hindrance, based on direct interference of antibody epitopes and homophilic binding sites.

Nevertheless, as shown in Figure 3, the EC5-specific 2G4 of the present invention is capable of inhibiting the homophilic Dsg3 trans-interaction, similarly to PV IgG. This indicates that beside steric hindrance, other forces such as allosteric conformational changes are capable of inducing loss of Dsg3-Dsg3 binding.

Beside the EC1 domain, 20% -50% of PV auto-ab bind to other epitopes in Dsg3 and may induce additional and synergistic effects. Nevertheless, it seems that only binding to defined epitopes induces allosteric conformational changes, because other Dsg3 specific antibodies, including EC5 specific AK9, exhibit no effect on Dsg3-Dsg3 interaction.

However, the interference of Dsg3 trans-interaction alone does not seem to be sufficient to induce a complete loss of adhesion. Desmosomes in Dsg3-knockout-mice are morphologically very similar to wildtype desmosomes and are capable of mediating cell adhesion. Furthermore, binding studies of PV-IgG antibodies to desmosomal Dsg3 at 4°C showed that Dsg3 binding alone is insufficient to induce loss of keratinocyte adhesion, and an incubation time of several hours is necessary to detect measurable changes in desmosomal structure.

Beside direct antibody impact on Dsg3 molecules, the activation of signalling pathways seems to represent another major component leading to acantholysis and blister formation. After antibody binding to keratinocytes, the activation of various signalling pathways like PLC/PKC, EFGR/Myc and p38MAPK could be observed.

Sarcoma associated kinase (Src) contributes to loss of cell adhesion mainly downstream of antibodies against Dsg3 in pemphigus. In terms of desmosomal integrity, the p38 MAPK pathway appears to be a key signalling mechanism, since selective inhibition of p38 prevents various PV features like cytoskeleton reorganisation, loss of adhesion and blister formation. Loss of keratinocyte adhesion was also abrogated when direct inhibition, as revealed by AFM on the surface of living keratinocytes, was still detectable.

The EC1-specific, pathogenic AK23 antibody is capable of inducing phosphorylation of p38 after binding to Dsg3, but, in comparison to PV IgG, the overall activation level of p38 appears significantly lower. Interestingly, inhibition of p38 prevents PV IgG induced but not AK23 mediated loss of adhesion, indicating two independent pathways. Nevertheless, activation of p38MAPK still represents a marker for the pathogenic potential of a Dsg3 specific antibody.

Therefore, the inventors' studies on the p38MAPK activation capacity of 2G4 show that binding of 2G4 to HaCaT cells, similar to AK23, subsequently activates p38MAPK, indicating that EC5 specific antibodies are also capable of inducing signal transduction in human keratinocytes. This observation is of crucial importance for the pathological impact of polyclonal PV IgG, where the antibodies exhibiting specificities against different subdomains seem to act synergistically.

The PV-associated internalisation of Dsg3 is probably caused by the induction of the described cell signalling events. It has been described previously that already within 60 min after adding PV IgG to human keratinocytes, non-desmosomal Dsg3 is endocytosed, and a rearrangement of desmosomal components in a linear structure occurs. Afterwards, these structures seem to act as sites for endocytosis of desmosomal Dsg3. By 3-6 hours, junctional Dsg3 is partly, and after 24 hours largely depleted from desmosome. Monoclonal antibodies alone exhibit a Dsg3-depleting activity that correlates with their pathogenicity.

In the inventors' study, binding of the 2G4 antibody also leads to a strong redistribution of Dsg3 after 24 h but, in comparison to Ak23, the intensity of linear array formation and Dsg3 endocytosis may be less distinct (Figure 4).

Finally, antibody binding in combination with signalling events, followed by Dsg3 depletion from the desmosome, leads to the disruption of desmosomal integrity and loss of adhesion, which is characterized by epidermal destabilization and intraepidermal blister formation.

As shown by keratinocyte dissociation assay, the antibody 2G4 of the invention significantly destabilizes keratinocyte adhesion. This was confirmed in an *ex vivo* model of human skin, where the 2G4 antibody induced acantholysis with a clear intraepidermal blister formation. This mirrors the pathogenic potential of 2G4 and shows that, in contrast to the current concept, EC5 specific antibodies may significantly contribute to the clinical phenotype of PV. In the present invention, higher concentrations of 2G4 had to be applied to obtain results comparable to Ak23, indicating a lesser pathogenic activity. This confirms that EC1-specific antibodies are the driving forces in the development of PV and represent the group of antibodies with the highest pathogenic potential. Nevertheless, the novel antibody 2G4 of the invention clearly shows that antibodies against other Dsg3 subdomains also have pathogenic potential.

As mentioned before, pathogenic EC5-specific molecules may play an important role concerning synergistic effects of polyclonal patient IgG containing EC1- and EC5-specific antibodies.

To summarize, the inventors demonstrate for the first time that a Dsg3-specific autoantibody directed against the membrane-proximal EC5 domain of desmoglein 3 exhibits the potential to induce similar pathogenic effects as described for amino-terminal EC1 specific antibodies like Ak23.

Thus, the novel antibody 2G4 of the invention represents a powerful tool to analyse the impact of membrane-proximal antibody binding to the Dsg3 molecule with regard to cell signalling events and the subsequent acantholysis induction. This invention highlights novel aspects for the understanding of the complex process of blister formation in PV and thereby contributes to the development of therapeutic strategies more independent from immunosuppressive medications.

A comparison of properties of the Dsg3-specific monoclonal antibodies 2G4 (invention) and Ak23 (known) is shown in the following Table 1.

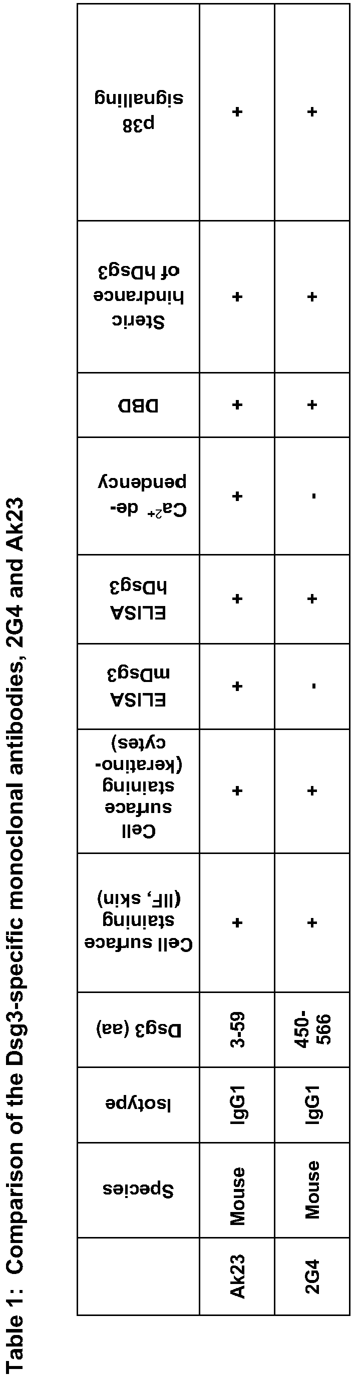

### Examples

### Abbreviations

- AFM =: atomic force microscopy
- ColVII NC1 =: Collagen VII, non-collagenous domain 1
- DBD =: dispase-based dissociation assay
- Dsg1 =: desmoglein 1
- Dsg3 =: desmoglein 3
- DMEM =: Dulbecco's Modified Eagle Medium
- EDTA =: Ethylenediaminetetraacetic acid
- EGFR =: Epidermal growth factor receptor
- ETA =: exfoliative toxin A
- GAPDH =: Glyceraldehyde-3-phosphate dehydrogenase
- hDsg =: human Dsg
- HRP =: Horseradish peroxidase
- MAPK =: Mitogen-activated protein kinases
- mDsg =: murine Dsg
- PBS =: phosphate-buffered saline
- PMS =: pooled murine serum
- PV =: Pemphigus vulgaris
- IF =: Immunofluorescence

### Cell culture and cell lines

The immortalized, non-tumorigenic human keratinocyte cell line HaCaT (CLS-300493, Cell lines Service, Eppelheim, Germany) was cultured in DMEM (Dulbecco's Modified Eagle Medium; Gibco, life technologies, Thermo Fisher Scientific Inc., USA) containing 10% FCS (fetal bovine serum, Biochrom AG, Berlin, Germany)/ Streptomycin (50 µg)-Penicillin (50 units/ml)-Glutamine(2 mM) in a humidified atmosphere containing 5% CO₂ at 37°C. Cells were seeded out with a density of 20 000 cells/cm² and passaged when they reached confluence of 80-90%.

### Protein production and purification

Full length Dsg3 (extracellular domain) and subdomains EC1-5 carrying an E-Tag were produced in baculovirus infected insect cells (High Five; Invitrogen, Carlsbad, CA, USA) as described previously (Müller R. et al., "IgG against extracellular subdomains of desmoglein 3 relates to clinical phenotype of pemphigus vulgaris", Experimental dermatology 2008;17(1):35-43; Rafei D. et al., "IgG autoantibodies against desmocollin 3 in pemphigus sera induce loss of keratinocyte adhesion", The American journal of pathology 2011;178(2):718-232011). The recombinant proteins were purified by affinity chromatography using nickel-nitrilotriacetic agarose beads (Qiagen, Hilden, Germany) according to manufacturer's instructions. Purified proteins were gradually dialysed against PBS supplemented with 0.5 mmol/L CaCl₂. Quality control was performed by Coomassie staining, ELISA and Western blotting.

### Antibody production and purification

Dsg3-(EC5)-specific monoclonal antibodies were purified from culture supernatants of B-cell hybridomas generated in HLA-DRA1*01:01-DRB1*04:02/-DQA1*03:01, - DQB1*03:02 (DQ8)-transgenic C57BI/6J mice expressing the human CD4 co-receptor and deficient for I-Aβ (I-Aβ^{-/-}) (Eming et al., 2014, a.a.O.). Mice were immunized with the human Dsg3 ectodomain and splenocytes were harvested five weeks later. Splenic B cells were isolated and fused with myeloma cells (Sp2/0-AG14) (https://www.atcc.org/products/tib-9) in a previously described hybridoma production procedure (Köhler G., Milstein C. "Continuous cultures of fused cells secreting antibody of predefined specificity". Nature 1975;256(5517):495-7).

Cells were cultured in HAT medium (based on RPMI medium, PAN Biotech, Aidenbach, Germany (Holliday R, Ho T. "Evidence for gene silencing by endogenous DNA methylation". Proceedings of the National Academy of Sciences of the United States of America 1998;95(15):8727-32), and supernatants were collected after seven days. Supernatants were filtered, and formed IgG antibodies were purified by affinity chromatography using protein G columns (GE-Healthcare, Munich, Germany) following standard protocols. The eluate was collected in a small amount of neutralisation buffer (1 M Tris-HCI, pH 9) followed by dialysis in PBS (Fig. 6).

### SDS Gel Electrophoresis and Western blot Analysis

Detection of mouse and human Dsg3 (full length extracellular domain) and human Dsg3 subdomains was performed by Western blot analysis according to a previous protocol (Müller et al. 2008, a. a. O.). For specific detection, primary murine antibodies (here: 2G4) (1:100) and a monoclonal rabbit anti-E-Tag antibody (1:7000; Abcam, Cambridge, UK) were used. Horseradish peroxidase (HRP)-conjugated anti-mouse IgG or anti-rabbit IgG (1:2000; Dako, Glostrup, Denmark) served as secondary antibodies. Antibody binding was visualized by using a commercial HRP substrate (Immobilon Western Chemiluminescent HRP substrate; Millipore, Billerica, MA). Signals were detected with a digital chemiluminescence reader (PEQLAB, Erlangen, Germany).

### Keratinocyte Dispase-based Dissociation Assay

Immortalized, non-tumorigenic human keratinocyte cells (HaCaTs, Cell lines Service, Eppelheim, Germany) were seeded into 12-well plates (Nunclon^{™} Surface, Nunc, Roskilde, Denmark) and grown to confluence in DMEM medium (Gibco, life technologies, Thermo Fisher Scientific Inc., USA). The cells were incubated with purified 2G4 antibodies (100 µg/ml), pooled control IgG (100 µg/ml) obtained from mice treated with PBS, anti-Dsg3 monoclonal ab Ak23, 2 µg/ml, provided by Dr. Amagai, Keio University Tokyo, Japan and Dr. Eliane Mueller, University Bern, Switzerland) in a humidified atmosphere containing 5% CO₂ at 37°C for 22 h. For cleavage of Dsg1, recombinant ETA (0.5 µg/ml) was added for the last two hours. After 2 washes with PBS, the adherent keratinocyte monolayer was incubated again (37°C, 5% CO₂) for 20 minutes with dispase I (1U/ml) (Sigma-Aldrich, Saint Louis, USA). The resulting non-adherent monolayers were washed with PBS and subjected to mechanical stress by pipetting five times with a 1 ml pipette. Fragments were fixed in 1 ml of a 10% formalin solution and stained with crystal violet. Photographs were taken with a digital camera (Canon EOS 550), and the fragments were counted as described before (Rafei et al. 2011, a.a.O.). Relative dissociation scores were calculated using the number of keratinocyte fragments in relation to the maximal number of cell fragments obtained by the positive control (Ak23).

### p38 MAPK analysis

For stimulation analysis of p38, HaCaT cells (see above) were grown to confluence in 6-well-plates (Nunc, Roskilde, Denmark) and starved with pure DMEM medium (not containing FCS or antibiotics) over night. Stimulation with different antibodies (Ak23 and 2G4 25 µg/ml) lasted 30 min at 37°C. After that cells were lysated with lysis buffer (50 mM Tris-HCI pH 7.4, 150 mM NaCl, 2 mM EDTA, 1% Nonidet P-40, also containing phosphatase inhibitor cocktail; Roche Applied Sciences, Penzberg, Germany), and protein concentrations of the lysates were determined by Bradford assay (https://www.bio-rad.com/de-de/product/quick-start-bradford-protein-assay?ID=5ec149ee-0cd1-468b-8651-a2fe9de6944d&WT.mc_id=170125005135&WT.srch=1&WT.knsh_id=75679249-45eb-45f4-a76f-f3fb9a39ffcb). 35µg/well of lysates were loaded on 10% SDS-gels to perform standard electrophoresis procedure as described previously (Rafei et al. 2011, a.a.O.). Phosphorylation of p38 MAP kinase was detected by Western blot analysis. Protein samples from SDS-PAGE were transferred onto nitrocellulose membranes and blocked for 30 min with 5% milk powder in TBS (TRIS-buffered saline) + 0.05% Tween-20 (TBS-T). As primary antibodies, phospho-p38 MAPK (D3F9, #4511, 1:1000, rabbit), p38 MAPK antibody (#9212; 1:1000, rabbit; both Cell Signaling Technology, Inc., Danvers, MA, USA) and GAPDH antibody (6C5, 1:30.000, mouse; Abcam, Cambridge, UK) were used.

### Enzyme linked immunosorbent assay (ELISA)

ELISA was performed as previously described (Müller R. et al., "IgG reactivity against non-conformational NH-terminal epitopes of the desmoglein 3 ectodomain relates to clinical activity and phenotype of pemphigus vulgaris", Experimental dermatology 2006;15(8):606-14). Briefly, full-length extracellular domain of human Dsg3 or recombinant ColVII NC1 were coated onto an immunomicrotitre plate (96-Well; Greiner Bio-one, Frickenhausen, Germany). As primary antibodies, 2G4 (30-60 µg/ml) or E-tag-antibody (1:2000) were used. For detection, species specific HRP conjugates antibodies (1:2000, Dako, Glostrup, Denmark) were used. Absorbance level was measured at 405 nm (Tecan plate reader Sunrise + Magellan software; Tecan Group Ltd., Männedorf, Switzerland).

For EDTA treatment, precoated ELISA plates (EUROIMMUN Dsg3 ELISA Kit, Lübeck, Germany) were incubated with 0.5 mmol/l EDTA for 30 min at room temperature (RT) as described before (Kamiya, K. et al., "Detection of antibodies against the non-calcium-dependent epitopes of desmoglein 3 in pemphigus vulgaris and their pathogenic significance", The British journal of dermatology 2012;167(2):252-61). After washing four times with Ca²⁺ free PBS, the ELISA was performed according to the manufacturer's protocol. Simultaneously, an ELISA without EDTA treatment was performed.

### Ex vivo model of human skin

4mm punch biopsies of human skin were taken from healthy individuals and incubated overnight (37°C, 5% CO₂) with 2G4 antibody (200-300 µg/ml) or mouse serum. For cleavage of Dsg1, 0.5 µg/ml exfoliative toxin A (ETA, Toxin Technology Inc., Sarasota, USA) was added to 2G4 and control IgG (pooled mouse serum). As standard procedure, paraffin-embedded skin-slides were stained with haematoxylin-eosin and images were taken by Olympus BH-2 (Olympus Cooperation, Tokyo, Japan). For IF staining, 2G4 or mouse serum were used as primary antibodies, and after 1 h incubation time visualised with species specific secondary antibodies labelled with fluorescein-5-isothiocyanat (FITC) (Rabbit anti-mouse IgG H&L (FITC) (ab6724), abcam).

### Passive transfer of Dsg3-specific IgG into neonatal mice

Passive transfer was performed using ca. 24-48 h old neonates (C57BI/6J, transgenic for a chimeric Dsg3 consisting of murine intracellular and transmembrane domain and a human extracellular Dsg3 domain). 100 µg of 2G4 or isotype control IgG (pooled mouse serum) in 25µl total volume supplemented with 1µg ETA, or PBS or ETA only, was injected subcutaneously into the nuchal fold. Mice were closely monitored and approx. 24 h later, the mice were sacrificed, tissues harvested and processed for routine histology and direct IF.

All animals were housed with 2 parental animals as one fresh litter per cage in a 12/12 h light/dark cycle with food and water available ad libitum. All experimental procedures were approved by the local animal ethics committee (G50/2018) and met German and international guidelines.

### Immunofluorescence (IF)

HaCaT cells (see above) were grown on coverslips until subconfluence in a 12 well plate (Nunclon^{™} Surface, Nunc, Roskilde, Denmark). Next, cells were incubated at 37°C for 1 h with either Ak23, mouse IgG or 2G4 as primary antibodies (25 µg/ml). Cells were then fixed with ice-cold methanol at -20°C for 5 min. After three washing steps with PBS, the bound primary antibodies were visualized with Alexa-488 labelled anti-mouse secondary antibody (1:300, from goat, Dianova, Pennsylvania, USA) or Cy3-labelled anti-mouse secondary antibody (1:300, goat, Jackson ImmunoResearch Europe Ltd, Suffolk, UK) that were incubated for 60 min at RT in the dark. Coverslips were mounted with Fluoromount (Dako, Glostrup, Denmark) on slides. Images were obtained by Olympus BH-2 microscope and analysed by CellD program (Olympus Cooperation, Tokyo, Japan).

### Confocal Microscopy

HaCaT cells (see above) were grown on coverslips in 12-well-plates (Nunclon^{™} Surface, Nunc, Roskilde, Danmark) until a monolayer was build. The monolayers were incubated with either Ak23 (10, 25 or 100 µg/ml), 2G4, or mouse IgG (both 100 µg/ml) for 24 h at 37°C. After fixation with ice cold methanol (-20°C, 5 min), the cells were incubated with 1% BSA/PBS (15 min, RT), followed by Dsg3 specific antibodies (5G11, anti-Dsg3 mouse antibody, Novus Biologicals, Littleton, USA, 1:100) for 60 min at RT. The secondary antibody treatment was carried out as above. Images were obtained with a confocal microscope (Zeiss LSM710 Confocal Laser Scanning Microscope, ZEN analysis program, Carl Zeiss, Oberkochen, Germany).

### Atomic force microscopy (AFM)

For AFM measurements, binding events between AFM cantilevers (MLCT, Bruker, Mannheim) and mica sheets, both functionalized with recombinant human Dsg3-Fc) was evaluated using a NanoWizard 3 AFM (Bruker) on an inverted optical microscope (Axio Observer, Carl Zeiss, Jena, Germany). The setup and approach were described in detail before (Vielmuth F. et al., "Atomic force microscopy identifies regions of distinct desmoglein 3 adhesive properties on living keratinocytes", Nanomedicine nanotechnology, biology, and medicine 2015a;11(3):511-20; Vielmuth F. et al., "Loss of Desmoglein Binding Is Not Sufficient for Keratinocyte Dissociation in Pemphigus", The Journal of investigative dermatology 2015b;135(12):3068-77). Generally, baseline (control) binding frequency was obtained, followed by a 30 min incubation with the respective antibodies at 37° C. Results of a second run at the same site of the same cantilever/mica combination were then compared to baseline and expressed as % of control. For each condition, at least three independent cantilever/mica combinations were evaluated.

### Statistical Analysis

Unless otherwise stated, all experiments were performed at least three times. For statistical analysis, Western blot bands of proteins were quantified by scanning densitometry using Image J (Freeware) and normalized to GAPDH. Data were analysed using commercially available software (SPSS). For multiple group comparisons, one-way ANOVA analysis of variance was used. Statistical significance was assumed at p < 0.05. Error bars in graphs represent standard deviation, mean ±SD.

**Electronic manipulation of images**

## Claims

1. A monoclonal IgG antibody 2G4 which
• is specific for the mebrane-proximal extracellular region EC5 of Desmoglein-3 designated as human Dsg3-EC5 (aa451-566)
• shows no cross-reaction with mouse Dsg3 or human Dsg1
• is capable of inhibiting the homophilic Dsg3-trans-interaction
• induces cellular Dsg3 redistribution by activation of the p38MAPK signal transduction pathway and
exhibits pathogenic Ca2+-independent binding to human Dsg3.

2. Monoclonal antibody 2G4 of claim 1 produced by a B-cell hybridoma deposited at DSMZ, Braunschweig under the accession number DSM ACC3371.

3. Process for producing the monoclonal antibody of any of claims 1 or 2, the process comprising
• immunizing HLA-DRA1*01:01-DRB1*04:02/-DQA1*03:01, -DQB1*03:03(DQ8) transgenic C57B1/6J mice expressing the human CD4 co-receptor and deficient for I-Aβ with the human Dsg3 ectdomain,
• harvesting splenocytes
• isolating splenic B cells
• fusing the isolated splenic B cells with myeloma cells Sp2/0-AG14
• culturing the hybridoma cells
• collecting and filtering supernatants
• isolating IgG antibodies from the collected supernatants
• purifying the antibodies by affinity chromatography.

4. Monoclonal antibody 2G4 of any of claims 1 or 2, obtainable by the process of claim 3.

5. Use of a monoclonal antibody 2G4 according to claims 1, 2 or 4 as an *in vitro* diagnostic for pemphigus vulgaris.

6. Use of the monoclonal antibody 2G4 of claim 5, wherein the diagnostic is a positive control of pemphigus vulgaris.

7. Use of the monoclonal antibody 2G4 of any of claims 1, 2 or 4 as a scientific tool for analyzing the molecular and/or cellular mechanism involved in pemphigus vulgaris.

8. B-cell hybridoma DSM ACC3371.
